# EUROPEAN PATENT SPECIFICATION

(11) **EP 3 813 790 B1**
(45) Date of publication and mention of the grant of the patent: **31.12.2025**
(21) Application number: 19740282.9
(22) Date of filing: 28.06.2019
(51) Int. Cl.: A61K 9/00, A61K 47/10, A61K 47/02

(54) **LOW ODOR OR NO ODOR INHALER DESICCANT POLYMER**
GERUCHSARMES ODER GERUCHSFREIES TROCKENMITTELPOLYMER
POLYMÈRE DÉSHYDRATANT D'INHALATEUR À FAIBLE ODEUR OU SANS ODEUR

(30) Priority: 29.06.2018 US 201862692641 P
(43) Date of publication of application: 05.05.2021
(73) Proprietor: CSP Technologies, Inc., Auburn, Alabama 36832 (US)
(72) Inventor: STRINGER, Thomas, Auburn, Alabama 36832 (US); ABRAMS, William, Auburn, Alabama 36832 (US); PETERS, Gary, Auburn, Alabama 36832 (US)
(74) Representative: Berggren Oy
(86) International application number: PCT/US2019/039741
(87) International publication number: WO 2020/006363

(56) References cited:
- WO-A1-2011/039196
- US-A1- 2007 286 814
- US-A1- 2016 039 955

## Description

### BACKGROUND OF THE INVENTION

### 1. FIELD OF INVENTION

This disclosure relates to inhalers, and more particularly to inhalers with desiccant that is configured to produce a reduced amount of volatile odors, as compared to existing desiccated inhalers. Optionally, the desiccant in the inhaler of the disclosed concept is odor free or substantially odor free.

### 2. DESCRIPTION OF RELATED ART

There are many items that are preferably stored, shipped and/or utilized in an environment that must be controlled and/or regulated. For example, in the moisture control field, containers and/or packages having the ability to absorb excess moisture trapped therein have been recognized as desirable. The control of moisture, oxygen, ethylene and other gaseous substances may be desirable in medical, electronics and food packaging applications.

Conventionally, desiccants, oxygen absorbers and other active agents have been used in raw form, e.g., as loose particulates housed in sachets or canisters within packaging, to control the internal environment of the package. For many applications, it is not desired to have such loosely stored active substances. To address this problem, the assignee of the present application had developed active entrained polymers comprising active agents, wherein such polymers can be extruded and/or molded into desired forms, e.g., container liners, plugs, film sheets, pellets and other such structures. Optionally, such active entrained polymers may include channeling agents, such as polyethylene glycol (PEG), which form channels between the surface of the entrained polymer and its interior to transmit a selected material (e.g., moisture) to the entrained active agent (e.g., desiccant to absorb the moisture). Entrained polymers may be two phase formulations (i.e., comprising a base polymer and active agent, without a channeling agent) or three phase formulations (i.e., comprising a base polymer, active agent and channeling agent). Entrained polymers are described, for example, in U.S. Pat. Nos. 5,911,937, 6,080,350, 6,124,006, 6,130,263, 6,194,079, 6,214,255, 6,486,231, 7,005,459, and U.S. Pat. Pub. No. 2016/0039955 In addition, WO 2011/039196 discloses use of a desiccant-entrained material, which comprises a channeling agent (PEG), a base polymer and a molecular sieve, to stabilize the fine particle mass of an inhalation drug formulation emitted by a pMDI closed container system.

Inhalers (e.g., without limitation, dry powder, aerosol, and/or metered dose) are one common type of drug delivery device that may employ desiccant-entrained polymers. However, one problem with known inhalers is that they often emit an odor that is unpleasant to users, thus making them unpleasant to use. The sources of the odor can be attributed to degradation of the materials used including polymeric materials during molding to form the inhaler, parts of the inhaler, or the materials used for the desiccant-entrained polymer. Thus, it is desirable to provide an improved inhaler having a desiccant-entrained polymer that does not emit and/or removes foul odors to users.

### BRIEF SUMMARY OF THE INVENTION

Accordingly, in one aspect, an inhaler is provided. The inhaler includes a body portion defining a flow path for the administration of medicament to a patient, and an entrained polymer in the flow path secured to or integral with the body portion. The entrained polymer has a monolithic material that includes a polypropylene base polymer prepared using a metallocene catalyst, an active agent, and a channeling agent. The entrained polymer is capable of adsorbing volatile organic compounds and gives off no detectable amount of volatile organic compounds.

Optionally, in any embodiment of an inhaler according to the disclosed concept, the entrained polymer releases no detectable volatile organic compounds at room temperature.

Optionally, in any embodiment of an inhaler according to the disclosed concept, the entrained polymer in a 16 gram quantity releases less than 4 ppm, optionally less than 2 ppm, optionally zero volatile organic compounds after heating at 60° C for up to 72 hours.

In an inhaler according to the invention, the active agent comprises 1) a molecular sieve desiccant; and 2) as an odor adsorbing material a hydrophilic zeolite.

Optionally, in any embodiment of an inhaler according to the disclosed concept, the entrained polymer is disposed in, or is in fluid communication with, a compartment of the body portion that houses the medicament.

Optionally, in any embodiment, the inhaler is dry powder inhaler, an aerosol inhaler, or a metered dose inhaler.

Optionally, in any embodiment of an inhaler according to the disclosed concept, the active agent is from 30% to 80%, optionally from 30% to 75%, optionally from 30% to 70%, optionally from 35% to 70%, optionally from 40% to 65%, optionally from 45% to 55%, by weight of the entrained polymer.

Optionally, in any embodiment of an inhaler according to the disclosed concept, the desiccant is from 15% to 79%, optionally from 15% to 75%, optionally from 15% to 70%, optionally from 15% to 60%, optionally from 15% to 50%, optionally from 30% to 70%, optionally from 30% to 60%, optionally from 30% to 50%, optionally from 40% to 70%, optionally from 40% to 60%, optionally from 45% to 70%, optionally from 45% to 60%, optionally from 45% to 55% by weight of the entrained polymer.

Optionally, in any embodiment of an inhaler according to the disclosed concept, the odor adsorbing material is from 1% to 15%, optionally from 1%to 12%, optionally from 1%to 10%, optionally from 1% to 8%, optionally from 4% to 12%, optionally from 4% to 8%, optionally from 6% to 12%, optionally from 6% to 10% by weight of the entrained polymer.

### BRIEF DESCRIPTION OF SEVERAL VIEWS OF THE DRAWINGS

The disclosed concept will be described in conjunction with the following drawings in which like reference numerals designate like elements and wherein:
Fig. 1 is a perspective view of a plug formed of an entrained polymer that may be deposited onto a package or drug delivery device according to the methods and apparatus ofthe disclosed concept;
Fig. 2 is a cross section taken along line 2-2 of Fig. 1;
Fig. 3 is a cross section similar to that of Fig. 2, showing a plug formed of another embodiment of an entrained polymer that may be deposited onto a package or drug delivery device according to the methods and apparatus of the disclosed concept;
Fig. 4 is a schematic illustration of an entrained polymer that may be used according to methods of the disclosed concept, in which the active agent is an absorbing or adsorbing material, e.g., a desiccant or oxygen scavenger;
Fig. 5 is a cross section of a container having a plug formed of an entrained polymer according to an optional aspect of the disclosed concept housed therein;
Fig. 6 is a cross section of a container similar to that of Fig. 5, in which the plug and the container are formed integrally;
Fig. 7 is a cross section of a container having a liner formed of an entrained polymer according to an optional aspect of the disclosed concept housed therein;
Fig. 8 is a cross sectional view of a sheet or film formed of an entrained polymer according to an optional aspect of the disclosed concept, formed integrally with a barrier layer;
Fig. 9 is a schematic illustration of a portion of an optional inhaler, according to one non-limiting embodiment of the disclosed concept; and
Fig. 10 is a graph showing volatile organic compound (VOC) concentration from a non-limiting embodiment of a desiccant formulation according to the disclosed concept (M3050-451) and a high VOC-emitting desiccant (M3003-451) that is not representative ofthe disclosed concept, with a non-desiccated polymer vial as acontrol.

### DETAILED DESCRIPTION OF PREFERRED EMBODIMENTS OF THE DISCLOSED CONCEPT

### Definitions

As used herein, the term "active" is defined as capable of acting on, interacting with or reacting with a selected material (e.g., moisture or oxygen) according to the disclosed concept. Examples of such actions or interactions may include absorption, adsorption orrelease of the selected material.

As used herein, the term "active agent" is defined as a material that (1) is immiscible with the base polymer and when mixed and heated with the base polymer and the channeling agent, will not melt, i.e., has a melting point that is higher than the melting point for either the base polymer or the channeling agent, and (2) acts on, interacts or reacts with a selected material. The term "active agent" may include but is not limited to materials that absorb, adsorb or release the selected material(s). Active agents according to the disclosed concept may be in the form of mineral p articles, but (unless otherwise claimed) the disclo sed concept should not be viewed as limited to mineral particulate active agents. Nevertheless, the disclosed concept is particularly suited for entrained polymers formed with an active agent that is mineral in form, for example molecular sieve or silica gel.

As used herein, the term "base polymer" is a polymer used to provide structure for the entrained polymer. The base polymer is preferably capable of being extruded or molded to form an entrained polymer comprising an active agent. The base polymer optionally has a gas transmission rate of a selected material that is substantially lower than, lower than or substantially equivalentto, that ofthe channeling agent. By way of example, such a transmission rate would be a water vapor transmission rate in embodiments where the selected material is moisture and the active agent is a water absorbing desiccant. The primary function of the base polymer is to provide structure for the entrained polymer. Suitable base polymers may include thermoplastic polymers, e.g., polyolefins such as polypropylene and polyethylene, polyisoprene, polybutadiene, polybutene, polysiloxane, polycarbonates, polyamides, ethylene-vinyl acetate copolymers, ethylene-methacrylate copolymer, poly(vinyl chloride), polystyrene, polyesters, polyanhydrides, polyacrylianitrile, polysulfones, polyacrylic ester, acrylic, polyurethane and polyacetal, or copolymers or mixtures thereof.

Referring to such a comparison of the base polymer and channeling agent water vapor transmission rate, in one embodiment, the channeling agent has a water vapor transmission rate of at least two times that of the base polymer. In another embodiment, the channeling agent has a water vapor transmission rate of at least five times that of the base polymer. In another embodiment, the channeling agent has a water vapor transmission rate of at least ten times that of the base polymer. In still another embodiment, the channeling agent has a water vapor transmission rate of at least twenty times that of the base polymer. In still another embodiment, the channeling agent has a water vapor transmission rate of at least fifty times that of the base polymer. In still another embodiment, the channeling agent has a water vapor transmission rate of at least one hundred times that of the base polymer.

As used herein, the term "channeling agent" or "channeling agents" is defined as a material that is immiscible with the base polymer and has an affinity to transport a gas phase substance at a faster rate than the base polymer. Optionally, a channeling agent is capable of forming channels through the entrained polymer when formed by mixing the channeling agent with the base polymer. Optionally, such channels are capable of transmitting a selected material through the entrained polymer at a faster rate than in solely the base polymer.

As used herein, the term "channels" or "interconnecting channels" is defined as passages formed of the channeling agent that penetrate through the base polymer and may be interconnected with each other.

As used herein, the term "entrained polymer" is defined as a monolithic material formed of at least a base polymer with an active agent and optionally also a channeling agent entrained or distributed throughout. An entrained polymer thus includes two-phase polymers and three phase polymers. An entrained polymer having an active agent that is mineral in form is referred to herein as a "mineral entrained polymer".

As used herein, the term "monolithic," "monolithic structure" or "monolithic composition" is defined as a composition or material that does not consist of two or more discrete macroscopic layers or portions. Accordingly, a "monolithic composition" does not include a multi-layer composite, but could optionally be part of such a component.

As used herein, the term "phase" is defined as a portion or component of a monolithic structure or composition that is uniformly distributed throughout, to give the structure or composition it's monolithic characteristics.

As used herein, the term "selected material" is defined as a material that is acted upon, by, or interacts or reacts with an active agent and is capable of being transmitted through the channels of the entrained polymer. For example, in embodiments in which a desiccant is used as an active agent, the selected material may be moisture or a gas that can be absorbed by the desiccant. In embodiments in which an adsorbing material is used as an active agent, the selected material may be certain volatile organic compounds and the adsorbing material may be activated carbon.

As used herein, the term "three phase" is defined as a monolithic composition or structure comprising three or more phases. An example of a three phase composition that may be used according to the disclosed concept would be an entrained polymer formed of a base polymer, active agent, and channeling agent. Optionally, a three phase composition or structure may include an additional phase, e.g., a colorant.

### Entrained Polymers Suitable for Methods According to the Disclosed Concept

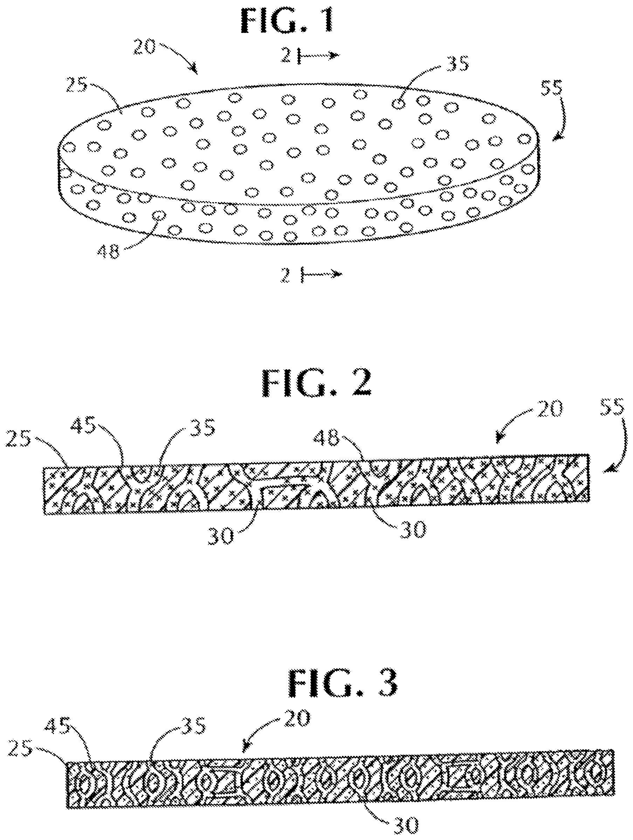

Figs. 1-8 illustrate entrained polymers 10 and various packaging assemblies or components of packaging assemblies or drug delivery devices formed of entrained polymers according to the disclosed concept. The entrained polymers 10 each include a base polymer 25, a channeling agent 35 and an active agent 30. As shown, the channeling agent 35 forms interconnecting channels 45 through the entrained polymer 10. At least some ofthe active agent 30 is contained within these channels 45, such that the channels 45 communicate between the active agent 30 and the exterior of the entrained polymer 10 via channel openings 48 formed at outer surfaces of the entrained polymer 10. The active agent 30 can be, for example, any one of a variety of absorbing or adsorbing materials, as described in further detail below. While a channeling agent, e.g., 35, is preferred, the disclosed concept broadly includes entrained polymers that optionally do not include channeling agents.

Suitable base polymers may include thermoplastic polymers, e.g., polyolefins such as polypropylene and polyethylene, polyisoprene, polybutadiene, polybutene, polysiloxane, polycarbonates, polyamides, ethylene-vinyl acetate copolymers, ethylene-methacrylate copolymer, poly(vinyl chloride), polystyrene, polyesters, polyanhydrides, polyacrylianitrile, polysulfones, polyacrylic ester, acrylic, polyurethane and polyacetal, or copolymers ormixture s thereof. In a preferred embodiment, a polyolefin such as polypropylene or polytheylene is used.

Suitable channeling agents may include a polyglycol such as polyethylene glycol (PEG), ethylene-vinyl alcohol (EVOH), polyvinyl alcohol (PVOH), glycerin polyamine, polyurethane and polycarboxylic acid including polyacrylic acid or polymethacrylic acid. Alternatively, the channeling agent 35 can be, for example, a water insoluble polymer, such as a propylene oxide polymerisate-monobutyl ether, such as Polyglykol B01/240, produced by CLARIANT. In other embodiments, the channeling agent could be a propylene oxide polymerisate monobutyl ether, such as PolyglykolB01/20, produced by CLARIANT, propylene oxide polymerisate, such as Polyglykol D01/240, produced by CLARIANT, ethylene vinyl acetate, nylon 6, nylon 66, or any combination of the foregoing.

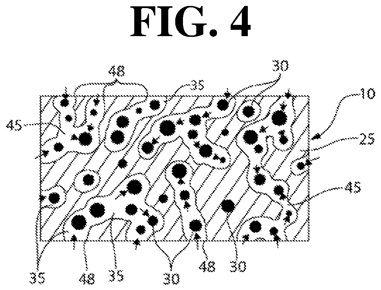

Suitable active agents according to the disclosed concept include absorbing or adsorbing materials, such as desiccating compounds and odor adsorbing substances. Fig. 4 illustrates an embodiment of an entrained polymer 10 according to the disclosed concept, in which the active agent 30 is an absorbing and/or adsorbing material. The arrows indicate the path of the selected material, for example moisture or gas, from an exterior of the entrained polymer 10, through the channels 45, to the particles of active agent 30, which absorb or adsorb the selected material.

If the active agent is a desiccant, any suitable desiccant for a given application may be used. Typically, physical absorption desiccants are preferred for many applications. These may include molecular sieves, silica gels, clays and starches. Alternatively, the desiccant may be a chemical compound that forms crystals containing water or compounds which react with water to form new compounds.

If the active agent is an odor adsorbing substance, any suitable microporous substance may be used. For example, charcoal, porous glass, clays, and aluminosilicate minerals including natural or synthetic zeolites. Some materials may be dual desiccants and odor adsorbing substances.

Optionally, in any embodiment, the active agent may be an oxygen scavenger.

Suitable absorbing materials may also include: (1) metals and alloys such as, but not limited to, nickel, copper, aluminum, silicon, solder, silver, gold; (2) metal-plated particulates such as silver-plated copper, silver-placed nickel, silver-plated glass microspheres; (3) inorganics such as BaTiO₃, SrTiO₃, SiO₂, Al₂O₃, ZnO, TiO₂, MnO, CuO, Sb₂O₃, WC, fused silica, fumed silica, amorphous fused silica, sol-gel silica, sol-gel titanates, mixed titanates, ion exchange resins, lithium-containing ceramics, hollow glass microspheres; (4) carbon-based materials such as carbon, activated charcoal, carbon black, ketchem black, diamond powder; (5) elastomers, such as polybutadiene, polysiloxane, and semi-metals, ceramic and; (6) other fillers and pigments.

In another example, the absorbing material may be a carbon dioxide scavenger, such as calcium oxide. In the presence of moisture and carbon dioxide, the calcium oxide is converted to calcium carbonate. Accordingly, calcium oxide may be used as the absorbing material in applications where absorption of carbon dioxide is needed. Such applications include preserving.

It is believed that the higher the active agent concentration in the mixture, the greater the absorption or adsorption capacity (as the case may be) will be of the final composition. However, too high an active agent concentration could cause the entrained polymer to be more brittle and the molten mixture of active agent, base polymer and channeling agent to be more difficult to either therm ally form, extrude or injection mold. In one embodiment, the active agent loading level can range from 10% to 80%, preferably 40% to 70%, more preferably from 40% to 60%, and even more preferably from 45% to 55% by weight with respect to the total weight of the entrained polymer. Optionally, channeling agent may be provided in a range of 2% to 10% by weight, preferably about 5%. Optionally, the base polymer may range from 10%to 50% by weight of the total composition, preferably from 20% to 35% by weight. Optionally, a colorant is added, e.g., at about 2% by weight of the total composition.

Referring to FIG. 1, an insert 20, constructed from the entrained polymer according to an optional embodiment is illustrated. The insert 20 is in the form of a plug 55 that may be deposited into a container, inhaler, drug delivery device, or other enclosure, or deposited onto a substrate (e.g., foil). Optionally, the insert 20 is integral with the substrate. For example, the insert 20 may be molded into the substrate in a two shot molding process or by another molding process. In a preferred embodiment, the substrate and the base polymer 25 are a similar or same material to facilitate the process.

Referring to FIG. 2, a cross-sectional view is shown of the plug 55 that has been constructed from an entrained polymer 10 comprising the base polymer 25 that has been uniformly blended with the active agent 30 and the hydrophilic agent or channeling agent 35. In the illustration of FIG. 2, the entrained polymer has been solidified so that interconnecting channels 45 have formed throughout the entrained polymer 10 to establish passages throughout the solidified plug 55. As may be appreciated from both FIGS. 1 and 2, the passages terminate in channel openings 48 at exterior surfaces of the plug 55. It should be noted that these illustrations are schematic in order to convey the different phases and the channels - not the actual size of these components and structures.

FIG. 3 illustrates the embodiment of a plug 55 similar in construction and makeup to the plug 55 of FIG. 2, where interconnecting channels 45 are very fine compared to those of FIG. 2. This can result from the use of a dimer agent (i.e., a plasticizer) together with a channeling agent 35. The dimer agent may enhance the compatibility between the base polymer 25 and the channeling agent 35. This enhanced compatibility is facilitated by a lowered viscosity of the blend, which may promote a more thorough blending of the base polymer 25 and channeling agent 35, which under normal conditions can resist combination into a uniform solution. Upon solidification of the entrained polymer 10 having a dimer agent added thereto, the interconnecting channels 45 which are formed therethrough have a greater dispersion and a smaller porosity, thereby establishing a greater density of interconnecting channels throughout the plug 55.

Interconnecting channels 45, such as those disclosed herein, facilitate transmission of a desired material, such as moisture, gas or odor, through the base polymer 25, which generally resists permeation of these materials, thus acting as a barrier thereto. For this reason, the base polymer 25 itself acts as a barrier substance within which an active agent 30 may be entrained. The interconnecting channels 45 formed of the channeling agent 35 provide pathways for the desired material to move through the entrained polymer 10. Without these interconnecting channels 45, it is believed that relatively small quantities of the desired material would be transmitted through the base polymer 25 to or from the active agent 30. In the case in which the desired material is transmitted to the active agent 30, it may be absorbed by the active agent 30, for example in embodiments in which the active agent 30 is an active agent such as a desiccant or an oxygen absorber.

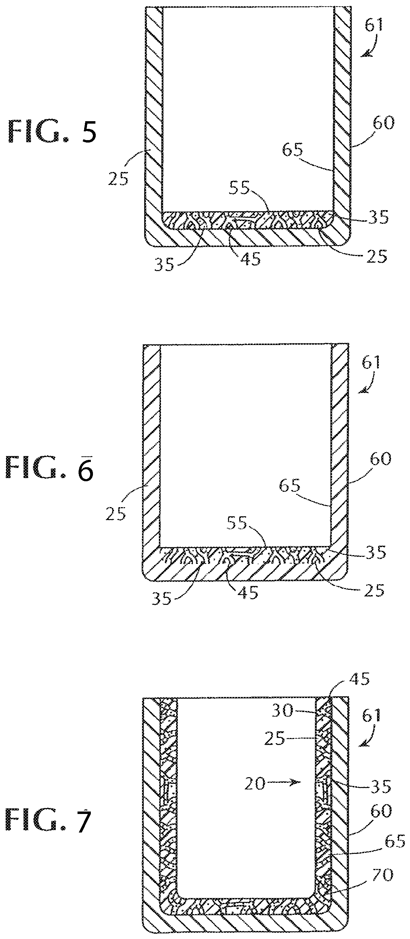

The entrained polymer 10 of the present invention has numerous applications. One exemplary application is the construction of rigid containers 61, which are suitable for containing relatively small volumes of product such as foodstuffs and medicines. In many cases, these types of products must be shipped and stored in controlled environments (e.g., reduced moisture and/or oxygen). It should be understood that the container 61 may also be representative of a compartment in an inhaler or other drug delivery device, for example, a compartment for storing medication to be delivered to a patient. In an embodiment, the entrained polymer 10 of the present invention may be formed into an insert for inclusion within the interior of the container 61. An example of one form of an insert is a plug 55 of any suitable shape, such as that shown in FIGS. 5 and 6. While the plug 55 would serve its purpose by being merely deposited within the container, it may also be fixed to an interior location so that it does move about within the interior space. The plug 55 may be formed into a disc that is shaped and sized to be press fitted snugly into a receiving location at the bottom of a polymeric container 61, as shown in FIGS. 5 and 6.

In other embodiments as shown in FIG. 7, a liner 70 may be formed from the entrained polymer 10, which has an exterior surface substantially conforming to an interior surface of the container body 60. Like the plug 55 described above, the liner 70 may be sized so as to be press-fit into position within the container body 60 where it is held sufficiently snugly to prevent unintended disengagement therefrom. Alternatively, either the plug 55 or liner 70 may be initially constructed and allowed to harden, and then the container body 60 subsequently constructed thereabout so that the greater shrinkage characteristics of the polymeric container body 60 not containing entrained polymer, cause the container body 60 to tightly shrink fit about the plug 55 or liner 70 so that neither becomes easily disengaged from the other. In still a further embodiment, the insert taking the form of either a plug 55 or a liner 70 may be simultaneously co-molded with the container body 60 so that each is integrally joined with the other. In embodiments formed by way of such co-molding, the viscosities of the entrained polymer 10 insert and the container body 60 may be approximately equal to facilitate the proper and desired location of the two phases of liquid or molten material that are molded together.

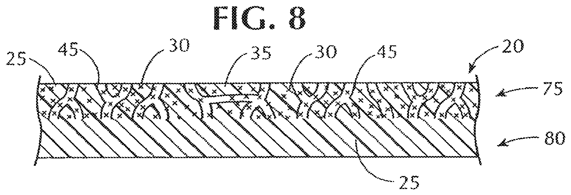

In yet another embodiment as shown in Fig. 8, two sheets or films 75, 80 are separately molded, and later combined to form a packaging wrap. Alternatively, the sheets or films 75, 80 may be coextruded. The packaging wrap comprising the two sheets 75, 80 as a composite provides active characteristics at an interior surface formed by the entrained polymer 10 active sheet 75 (also designated as "20" consistent with the reference numeral corresponding to the insert 20 as discussed in the above-described embodiments), and vapor resistant or gas impermeable characteristics at an exterior surface formed by the barrier sheet 80. Optionally, this composite of sheets 75, 80 may be incorporated into a drug delivery device according to an optional aspect of the disclosed concept.

### Drug Delivery Device According to the Disclosed Concept

The disclosed concept provides a drug delivery device, optionally an inhaler, including an entrained polymer positioned in the flow path of the administered drug. Optionally, the administered drug is in the form of a solid. The entrained polymer comprises a monolithic material comprising a base polymer, an active agent, and optionally a channeling agent. Optionally, the active agent comprises a desiccant and a microporous odor adsorbing material. Optionally, the desiccant is a molecular sieve. Optionally, the microporous odor adsorbing material is a zeolite. Optionally, the zeolite is a hydrophilic zeolite.

The disclosed concept is described in greater detail below using an inhaler as an example of the drug delivery device.

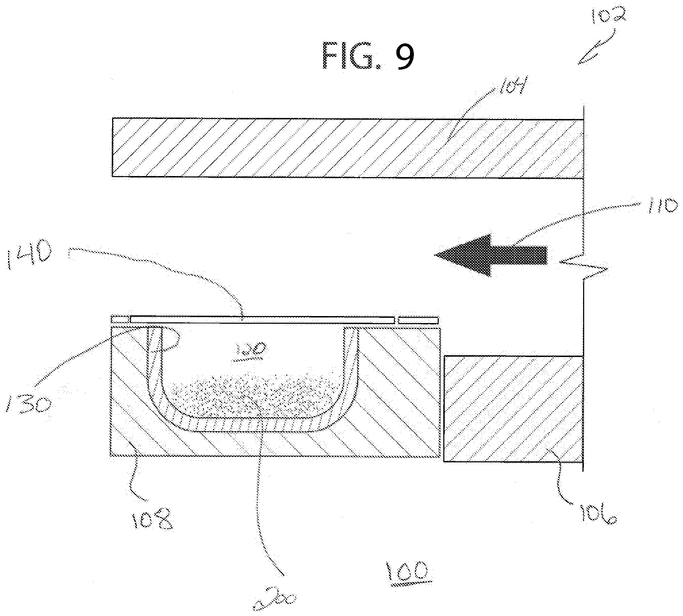

FIG. 9 is a schematic illustration of a portion of an inhaler 100, according to one non-limiting embodiment of the disclosed concept. The inhaler 100 may be, for example and without limitation, a dry powder inhaler, an aerosol inhaler, or a metered dose inhaler. In the example shown, the inhaler 100 is a dry powder inhaler, but the disclosed concept is not limited thereto.

The inhaler 100 includes a body portion 102 optionally comprising atop portion 104, a bottom portion 106, and a main housing portion 108. The body portion 102 defines a flow path 110 for the administration of medicament 200 (*e.g.*, without limitation, a medicine) to a patient. The main housing portion 108 has a compartment 120 configured to contain a predetermined quantity of the medicament 200. As shown, the inhaler 100 further has an entrained polymer 130 which is optionally a desiccant-entrained polymer. In the exemplary embodiment the entrained polymer 130 is secured to the main housing portion 108. However,it will be appreciated that in a suitable alternative inhaler (not shown), an entrained polymer may be integral with the body portion, without departing from the scope of the disclosed concept.

Continuing to refer to FIG. 9, the entrained polymer 130 is located in (*e.g.*, and/oris in fluid communication with) the compartment 120 of the body portion 102 that houses the medicament 200. As shown, the inhaler 100 further optionally includes a sealing element (*e.g.*, without limitation, a foil member 140) coupled to the main housing portion 108 in order to seal the medicament 200 in the compartment 120. It will thus be appreciated that when the foil member 140 is removed or punctured by a user, the medicament 200 can be inhaled by the user. Accordingly, the entrained polymer 130 is configured to be located in the flow path 110. Furthermore, in accordance with the disclosed concept, the inhaler 100 is advantageously configured so as to give off significantly lower amounts of volatile odors to a user (even as low as zero detectable volatile organic compounds or "VOCs"), as compared to prior art inhalers.

Specifically, prior art inhalers (not shown) which include entrained polymers, have entrained polymers that give off relatively high levels of volatile organic compounds, thus providing an overall unpleasant experience (taste and/or smell) for the user. However, in one example embodiment ofthe disclosed concept, the concentration of volatile organic compound given off by the entrained polymer 130 of the inhaler 100 preferably is less than 40 parts per million, more preferably less than 25 parts per million, more preferably less than 4 parts per million, optionally less than 2 parts per million, optionally less than 1 part per million, optionally zero detectable parts per million. As such, it will be appreciated that when the user removes the foil member 140 and inhales the medicament 200, a significantly more pleasant odor will be present, as compared to prior art inhalers.

The entrained polymer 130 preferably includes a monolithic material that has a base polymer, an active agent, and optionally a channeling agent. In one example embodiment the entrained polymer 130 includes the channeling agent. The active agent may be a desiccant, optionally a molecular sieve, a silica gel, a clay, or another desiccant that is a granular material that is more abrasive than the base polymer. The active agent may also comprise an odor adsorbing material, optionally a microporous material including a zeolite. The active agent in total (e.g., desiccant and odor adsorber combined) may be from 20% to 80%, optionally from 30% to 75%, optionally from 30%to 70%, optionally from 35%to 70%, optionally from 40%to 65%, optionally from 45% to 55%, by weight of the entrained polymer 130. The desiccant may be from 15% to 79%, optionally from 15% to 75%, optionally from 15% to 70%, optionally from 15% to 60%, optionally from 15% to 50%, optionally from 30% to 70%, optionally from 30%to 60%, optionally from 30%to 50%, optionally from 40%to 70%, optionally from 40%to 60%, optionally from 45% to 70%, optionally from 45% to 60%, optionally from 45% to 55% by weight of the entrained polymer 130. The odor adsorbing material may be from 1%to 15%, optionally from 1% to 12%, optionally from 1% to 10%, optionally from 1% to 8%, optionally from 4% to 12%, optionally from 4% to 8%, optionally from 6% to 12%, optionally from 6% to 10% by weight of the entrained polymer 130.

Additionally, in one example embodiment the base polymer and/or the channeling agent are prepared using a metallocene catalyst. In another embodiment, the desiccant is combined with an odor absorbing zeolite. An example of the odor absorbing zeolite is a hydrophilic adsorbent such as that sold by ZEOCHEM under the name ZEOflair^{®} 1000. As a result, the entrained polymer 130 is significantly cleaner than traditional prior art entrained polymers (not shown), which typically retain their catalysts after processing. Moreover, the Metallocene is cleaner because it is easier to remove from the entrained polymer 130 during processing, as compared to prior art entrained polymers wherein their catalysts typically get stuck in the entrained polymers during processing. The Metallocene also advantageously lowers the energy requirement to form the entrained polymer 130.

In another aspect, the disclosed concept also provides a method of manufacturing a low or no odor inhaler. The method comprises providing a body portion defining a flow path for an administration of amedicamentto a patient, and an entrained polymer located in the flow path and being secured to or integral with the body portion, wherein the entrained polymer comprising a monolithic material that includes a base polymer, an active agent, and a channeling agent, and further wherein the entrained polymer at room temperature is odor free or at least substantially odor free. In an optional embodiment, the entrained polymer (in about 16 g quantity in a volume about 166 mL) at room temperature gives rise to a volatile organic compound concentration ofless than 4 parts per million, optionally less than 2 parts per million, optionally less than 1 parts per million, preferably 0 parts per million as measured by detection equipment as described herein. In an optional embodiment, the entrained polymer (in about 16 g quantity in a volume about 166 mL) after heating at 60° C for 72 hours gives rise to a volatile organic compound concentration of less than 40 parts per million, preferably less than 25 parts per million, more preferably less than 4 parts per million, more preferably less than 2 parts per million, most preferably 0 parts per million. The material of the body portion or any portion of the inhaler is not particularly limited.

In another aspect, the disclosed concept provides an inhaler for use in a method of administering a medicament to a patient in need thereof, the method comprising the step of delivering the medicament in an inhaler, wherein the inhaler comprises: a body portion defining a flow path for the an administration of a medicament to a patient; and an entrained polymer located in the flow path and being secured to or integral with the body portion, wherein the entrained polymer comprising a monolithic material that includes a polypropylene base polymer, an active agent, and a channeling agent, and further wherein the entrained polymer gives rise to a volatile organic compound concentration of less than 40 parts per million, preferably less than 25 parts per million, more preferably less than 4 parts per million even more preferably 0 parts per million as measured by detection equipment as described herein.

### EXAMPLES

### Example 1

Desiccant-entrained polymer resins M-3050 and M-3057 were prepared according to the following:

| Component | Function | M-3050 (%) | M-3057 (%) |
|---|---|---|---|
| Molecular Sieve 4A | Desiccant | 58.93 | 60 |
| Poly(ethylene) glycol (Carbowax 4000P) | Channeling agent | 5 | 5 |
| Irganox 225 (Phenol, 2,4-bis(1,1-dimethylethyl)-, phosphite and Benzenepropanoic acid, 3,5-bis(1,1-dimethylethyl)-4-hydroxy-,2-2-bis[[3-[3,5-bis(1,1-dimethylethyl)-4-hydroxyphenyl]-1-oxopropoxy] in 3:1 | Heat stabilizer | 0.08 | 0 |
| Polypropylene (Achieve 1605PP) | Base polymer | 26 | 25 |
| Titanium Dioxide | Colorant | 2 | 2 |
| ZEOFLARE 1000 | Adsorbent | 8 | 8 |

A reference desiccant-entrained polymer M-3003 was also prepared according to the following:

| Component | Function | Content (%) |
|---|---|---|
| Molecular Sieve 4A | Desiccant | 69 |
| Poly(ethylene) glycol | Channeling agent | 5 |
| Polypropylene | Base polymer | 24 |
| Titanium Dioxide | Colorant | 2 |

### Example 2

The desiccant-entrained polymers M-3050, M-3057 and reference polymer M-3003 were each used as the material for a sleeve in a vial, and extrusion molded into the vial to form a sleeved vial M-3050-451, M-3057-451 and M-3003-451,respectively. The dimensions of the vials and the sleeves are the following:

| Dimensions | Typical Value |
|---|---|
| Volume | 166 mL |
| Vial Overall Height | 101 mm |
| Vial Inside Height | 96 mm |
| Vial Outside Diameter | 52 mm |
| Sleeve Inside Diameter | 47 mm |

### Example 3

Three vials of each of M-3050-451 (sleeve weight ~16 grams) and M-3003-451 (sleeve weight ~16 grams) as well as one vial with no desiccant entrained polymer as a blank were each sealed with aluminum crimp cap, placed in 140 °F (60 °C) oven overnight. The concentration of the volatile organic compounds (VOC) as a measure of the odor emitted from the vials was measured by analyzing the gas in the headspace of each of the vials by GCMS with a photoionization detector.

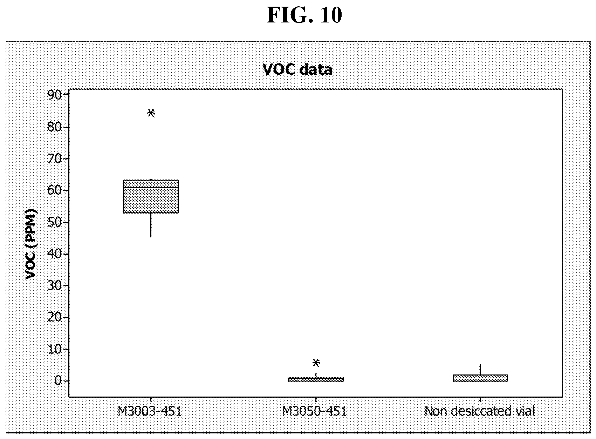

The results are shown in Figure 10. The odor emitted from the vial with the optional inventive desiccant-entrained polymer sleeve is significantly less than from a reference vial, and is comparable to the non-desiccated vial.

A smell test by a human subject revealed that the blank sample (non-desiccated vial) was slightly more odorous than the M-3050-451 vials, which emitted very faint to no odor (i.e., was odor free or substantially odor free).

### Example 4

Five sleeved vials of each of M-3050-451, M-3057-451 and M-3003-451 stored in a closed configuration at room temperature, or at room temperature followed by heating at 140 °F (60 °C) for 72 hrs, were evaluated for internal released VOC concentration. A puncturing apparatus with tubing connected to a syringe was used to puncture the lid of the vial. The gas in the headspace of each of the vials was collected using the syringe (65 mL) via the connected tubing; and was injected directly into a PHOTOVAC 2020 COMBO PRO gas analyzer. The VOC concentrations (average) in the internal cavity are as in the table below.

| | VOC Concentration (ppm) (room temperature) | VOC Concentration (ppm) (room temperature and heat) |
|---|---|---|
| M-3003-451 | 0.88 | 2.68 |
| M-3050-451 | 0 | 0.26 |
| M-3057-451 | 0 | 0.2 |

A smell test by a human subject revealed that the M-3003-451 vials were significantly more odorous than the M-3050-451 or M-3057-451 vials which emitted no detectable odor at room temperature or after heating.

The VOC and the smell tests here demonstrate that the optional inventive resin is a low or no odor desiccant-entrained resin for incorporation into inhalers.

## Claims

1. An inhaler comprising:
a body portion defining a flow path for an administration of a medicament to a patient; and
an entrained polymer located in the flow path and secured to or integral with the body portion, wherein the entrained polymer comprises a monolithic material comprising a polypropylene base polymer prepared using a metallocene catalyst, an active agent comprising a molecular sieve desiccant, a hydrophilic zeolite odor adsorbing material, and a channeling agent, and further wherein the entrained polymer is substantially odor free.

2. An inhaler comprising:
a body portion defining a flow path for an administration of a medicament to a patient; and
an entrained polymer located in the flow path and secured to or integral with the body portion, wherein the entrained polymer comprises a monolithic material comprising a polypropylene base polymer prepared using a metallocene catalyst, an active agent comprising a molecular sieve desiccant, a hydrophilic zeolite odor adsorbing material, and a channeling agent, and further wherein the entrained polymer in a 16 gram quantity releases less than 4 ppm volatile organic compounds after heating at 60° C for up to 72 hours.

3. The inhaler according to claim 1 or claim 2, wherein the entrained polymer releases no detectable volatile organic compounds at room temperature.

4. The inhaler according to claim 1, wherein the entrained polymer in a 16 gram quantity releases less than 4 ppm volatile organic compounds after heating at 60° C for up to 72 hours.

5. The inhaler according to any one of claims 1-4, wherein the inhaler is a dry powder inhaler.

6. The inhaler according to any one of claims 1-4, wherein the inhaler is an aerosol inhaler.

7. The inhaler according to any one of claims 1-4, wherein the inhaler is a metered dose inhaler.

8. The inhaler according to any one of claims 1-7, wherein the entrained polymer is disposed in, or is in fluid communication with, a compartment of the body portion that houses the medicament.

9. The inhaler according to any one of claims 1-8, wherein the active agent is from 30% to 80%, optionally from 30% to 75%, optionally from 30% to 70%, optionally from 35% to 70%, optionally from 40% to 65%, optionally from 45% to 55%, by weight of the entrained polymer.

10. The inhaler according to any one of claims 1-9, wherein the desiccant is from 15% to 79%, optionally from 15% to 75%, optionally from 15% to 70%, optionally from 15% to 60%, optionally from 15% to 50%, optionally from 30% to 70%, optionally from 30% to 60%, optionally from 30% to 50%, optionally from 40% to 70%, optionally from 40% to 60%, optionally from 45% to 70%, optionally from 45% to 60%, optionally from 45% to 55% by weight of the entrained polymer.

11. The inhaler according to any one of claims 1-10, wherein the odor adsorbing material is from 1% to 15%, optionally from 1% to 12%, optionally from 1% to 10%, optionally from 1% to 8%, optionally from 4% to 12%, optionally from 4% to 8%, optionally from 6% to 12%, optionally from 6% to 10% by weight of the entrained polymer.

12. A method of manufacturing a low odor inhaler, comprising the step of
providing a body portion defining a flow path for an administration of a medicament to a patient, and an entrained polymer located in the flow path and being secured to or integral with the body portion,
wherein the entrained polymer comprises a monolithic material that includes a polypropylene base polymer prepared using a metallocene catalyst, an active agent comprising a molecular sieve desiccant, a hydrophilic zeolite odor adsorbing material, and a channeling agent, and
further wherein the entrained polymer is odor free, or substantially odor free.

13. An inhaler for use in a method of administering a medicament to a patient in need thereof, the method comprising the step of delivering the medicament in an inhaler, wherein the inhaler comprises:
a body portion defining a flow path for an administration of a medicament to a patient; and
an entrained polymer located in the flow path and being secured to or integral with the body portion, wherein the entrained polymer comprises a monolithic material that includes a polypropylene base polymer prepared using a metallocene catalyst, an active agent comprising a molecular sieve desiccant, a hydrophilic zeolite odor adsorbing material, and a channeling agent, and further wherein the entrained polymer is odor free, or substantially odor free.

## Patentansprüche

1. Inhalator, welcher Folgendes umfasst:
einen Körperteil, der einen Strömungsweg für die Verabreichung eines Medikaments an einen Patienten definiert; und
ein mitgenommenes Polymer, das sich im Strömungsweg befindet und an dem Körperteil befestigt oder mit diesem einstückig ist, wobei das mitgenommene Polymer ein monolithisches Material umfasst, das ein Polypropylen-Grundpolymer, das unter Verwendung eines Metallocen-Katalysators hergestellt wurde, ein aktives Mittel, das ein Molekularsieb-Trockenmittel, ein hydrophiles Zeolith-Geruchsadsorptionsmaterial und ein Kanalisierungsmittel, und wobei ferner das mitgenommene Polymer im Wesentlichen geruchsfrei ist.

2. Inhalator, welcher Folgendes umfasst:
einen Körperteil, der einen Strömungsweg für die Verabreichung eines Medikaments an einen Patienten definiert; und
ein mitgenommenes Polymer, das sich im Strömungsweg befindet und an dem Körperteil befestigt oder mit diesem einstückig ist, wobei das mitgenommene Polymer ein monolithisches Material umfasst, das ein Polypropylen-Grundpolymer, das unter Verwendung eines Metallocen-Katalysators hergestellt wurde, ein aktives Mittel, das ein Molekularsieb-Trockenmittel, ein hydrophiles Zeolith-Geruchsadsorptionsmaterial und ein Kanalisierungsmittel, und wobei ferner das mitgenommene Polymer in einer Menge von 16 Gramm weniger als 4 ppm flüchtige organische Verbindungen nach dem Erhitzen auf 60°C für bis zu 72 Stunden freisetzt.

3. Inhalator nach Anspruch 1 oder Anspruch 2, wobei das mitgenommene Polymer bei Raumtemperatur keine nachweisbaren flüchtigen organischen Verbindungen freisetzt.

4. Inhalator nach Anspruch 1, wobei das mitgenommene Polymer in einer Menge von 16 Gramm weniger als 4 ppm flüchtige organische Verbindungen nach Erhitzen auf 60°C für bis zu 72 Stunden freisetzt.

5. Inhalator nach einem der Ansprüche 1-4, wobei der Inhalator ein Trockenpulver-Inhalator ist.

6. Inhalator nach einem der Ansprüche 1-4, wobei der Inhalator ein Aerosol-Inhalator ist.

7. Inhalator nach einem der Ansprüche 1-4, wobei der Inhalator ein Dosier-Inhalator ist.

8. Inhalator nach einem der Ansprüche 1 bis 7, wobei das mitgenommene Polymer in einem Fach des Körperteils, in dem das Medikament untergebracht ist, angeordnet ist oder mit diesem in Fluidverbindung steht.

9. Inhalator nach einem der Ansprüche 1-8, wobei der Wirkstoff 30 % bis 80 %, gegebenenfalls 30 % bis 75 %, gegebenenfalls 30 % bis 70 %, gegebenenfalls 35 % bis 70 %, gegebenenfalls 40 % bis 65 %, gegebenenfalls 45 % bis 55 %, bezogen auf das Gewicht des mitgenommenen Polymers, ausmacht.

10. Inhalator nach einem der Ansprüche 1 bis 9, wobei das Trockenmittel 15 % bis 79 %, gegebenenfalls 15 % bis 75 %, gegebenenfalls 15 % bis 70 %, gegebenenfalls 15 % bis 60 %, gegebenenfalls 15 % bis 50 %, gegebenenfalls 30 % bis 70 %, gegebenenfalls 30 % bis 60 %, gegebenenfalls 30 % bis 50 %, gegebenenfalls 40 % bis 70 %, gegebenenfalls 40 % bis 60 %, gegebenenfalls 45 % bis 70 %, gegebenenfalls 45 % bis 60 %, gegebenenfalls 45 % bis 55 % des Gewichts des mitgenommenen Polymers, ausmacht.

11. Inhalator nach einem der Ansprüche 1-10, wobei das geruchsadsorbierende Material 1 % bis 15 %, gegebenenfalls 1 % bis 12 %, gegebenenfalls 1 % bis 10 %, gegebenenfalls 1 % bis 8 %, gegebenenfalls 4 % bis 12 %, gegebenenfalls 4 % bis 8 %, gegebenenfalls 6 % bis 12 %, gegebenenfalls 6 % bis 10 %, bezogen auf das Gewicht des mitgenommenen Polymers, ausmacht.

12. Verfahren zur Herstellung eines geruchsarmen Inhalators, welches den folgenden Schritt umfasst
Bereitstellen eines Körperteils, der einen Strömungsweg für die Verabreichung eines Medikaments an einen Patienten definiert, und eines mitgenommenen Polymers, das sich in dem Strömungsweg befindet und an dem Körperteil befestigt oder mit diesem integriert ist,
wobei das mitgenommene Polymer ein monolithisches Material umfasst, das ein Polypropylen-Grundpolymer, das unter Verwendung eines Metallocen-Katalysators hergestellt wurde, ein aktives Mittel, das ein Molekularsieb-Trockenmittel umfasst, ein hydrophiles Zeolith-Geruchsadsorptionsmaterial und ein Kanalisierungsmittel, enthält, und
wobei das mitgenommene Polymer geruchsfrei oder im Wesentlichen geruchsfrei ist.

13. Inhalator zur Verwendung in einem Verfahren zur Verabreichung eines Medikaments an einen Patienten, der dieses benötigt, wobei das Verfahren den Schritt der Abgabe des Medikaments in einen Inhalator umfasst, wobei der Inhalator Folgendes umfasst:
einen Körperteil, der einen Strömungsweg für die Verabreichung eines Medikaments an einen Patienten definiert; und
ein mitgenommenes Polymer, das sich im Strömungsweg befindet und an dem Körperteil befestigt oder mit diesem einstückig ist, wobei das mitgenommene Polymer ein monolithisches Material umfasst, das ein Polypropylen-Grundpolymer, das unter Verwendung eines Metallocen-Katalysators hergestellt wurde, ein aktives Mittel, das ein Molekularsieb-Trockenmittel, ein hydrophiles Zeolith-Geruchsadsorptionsmaterial und ein Kanalisierungsmittel, und wobei ferner das mitgenommene Polymer geruchsfrei oder im Wesentlichen geruchsfrei ist.

## Revendications

1. Inhalateur comprenant :
une partie du corps définissant un trajet de flux pour une administration d'un médicament à un patient ; et
un polymère entraîné situé dans le trajet de flux et fixé à ou faisant partie intégrante de la partie du corps, dans lequel le polymère entraîné comprend un matériau monolithique comprenant un polymère de base en polypropylène préparé à l'aide d'un catalyseur métallocène, un agent actif comprenant un déshydratant à tamis moléculaire, un matériau zéolithique hydrophile adsorbant les odeurs et un agent de canalisation, et dans lequel en outre le polymère entraîné est sensiblement inodore.

2. Inhalateur comprenant :
une partie du corps définissant un trajet de flux pour une administration d'un médicament à un patient ; et
un polymère entraîné situé dans le trajet de flux et fixé à ou faisant partie intégrante de la partie corporelle, dans lequel le polymère entraîné comprend un matériau monolithique constitué d'un polymère de base en polypropylène préparé à l'aide d'un catalyseur métallocène, d'un agent actif comprenant un déshydratant à tamis moléculaire, un matériau zéolithique hydrophile adsorbant les odeurs et un agent de canalisation, et dans lequel en outre, le polymère entraîné, en quantité de 16 grammes, libère moins de 4 ppm de composés organiques volatils après un chauffage à 60 °C pendant une durée maximale de 72 heures.

3. Inhalateur selon la revendication 1 ou la revendication 2, dans lequel le polymère entraîné ne libère aucun composé organique volatil détectable à température ambiante.

4. Inhalateur selon la revendication 1, dans lequel le polymère entraîné en quantité de 16 grammes libère moins de 4 ppm de composés organiques volatils après un chauffage à 60 °C pendant une durée maximale de 72 heures.

5. Inhalateur selon l'une quelconque des revendications 1 à 4, dans lequel l'inhalateur est un inhalateur de poudre sèche.

6. Inhalateur selon l'une quelconque des revendications 1 à 4, dans lequel l'inhalateur est un inhalateur d'aérosol.

7. Inhalateur selon l'une quelconque des revendications 1 à 4, dans lequel l'inhalateur est un inhalateur à dose mesurée.

8. Inhalateur selon l'une quelconque des revendications 1 à 7, dans lequel le polymère entraîné est disposé dans, ou est en communication fluidique avec, un compartiment de la partie du corps qui abrite le médicament.

9. Inhalateur selon l'une quelconque des revendications 1 à 8, dans lequel l'agent actif représente de 30 % à 80 %, éventuellement de 30 % à 75 %, éventuellement de 30 % à 70 %, éventuellement de 35 % à 70 %, éventuellement de 40 % à 65 %, éventuellement de 45 % à 55 %, en poids du polymère entraîné.

10. Inhalateur selon l'une quelconque des revendications 1 à 9, dans lequel le déshydratant représente de 15 % à 79 %, éventuellement de 15 % à 75 %, éventuellement de 15 % à 70 %, éventuellement de 15 % à 60 %, éventuellement de 15 % à 50 %, éventuellement de 30 % à 70 %, éventuellement de 30 % à 60 %, éventuellement de 30 % à 50 %, éventuellement de 40 % à 70 %, éventuellement de 40 % à 60 %, éventuellement de 45 % à 70 %, éventuellement de 45 % à 60 %, éventuellement de 45 % à 55 % en poids du polymère entraîné.

11. Inhalateur selon l'une quelconque des revendications 1 à 10, dans lequel le matériau adsorbant les odeurs représente de 1 % à 15 %, éventuellement de 1 % à 12 %, éventuellement de 1 % à 10 %, éventuellement de 1 % à 8 %, éventuellement de 4 % à 12 %, éventuellement de 4 % à 8 %, éventuellement de 6 % à 12 %, éventuellement de 6 % à 10 % en poids du polymère entraîné.

12. Procédé de fabrication d'un inhalateur à faible odeur, comprenant l'étape suivante
la fourniture d'une partie du corps définissant un trajet de flux pour l'administration d'un médicament à un patient, et un polymère entraîné situé dans le trajet de flux et fixé à ou faisant partie intégrante de la partie du corps,
dans lequel le polymère entraîné comprend un matériau monolithique qui comporte un polymère de base en polypropylène préparé à l'aide d'un catalyseur métallocène, un agent actif comprenant un déshydratant à base de tamis moléculaire, un matériau zéolithique hydrophile adsorbant les odeurs, et un agent de canalisation, et
en outre, dans lequel le polymère entraîné est inodore, ou pratiquement inodore.

13. Inhalateur destiné à être utilisé dans un procédé d'administration d'un médicament à un patient qui en a besoin, le procédé comprenant l'étape d'acheminement du médicament dans un inhalateur, dans lequel l'inhalateur comprend :
une partie du corps définissant un trajet de flux pour l'administration d'un médicament à un patient ; et
un polymère entraîné situé dans le trajet de flux et fixé à ou faisant partie intégrante de la partie corporelle, dans lequel le polymère entraîné comprend un matériau monolithique qui comporte un polymère de base en polypropylène préparé à l'aide d'un catalyseur métallocène, un agent actif comprenant un déshydratant à tamis moléculaire, un matériau zéolithique hydrophile adsorbant les odeurs et un agent de canalisation, et dans lequel en outre le polymère entraîné est inodore ou sensiblement inodore.
